# EUROPÄISCHE PATENTANMELDUNG

(11) **EP 0 940 396 A1**
(43) Veröffentlichungstag der Anmeldung: **08.09.1999**
(21) Anmeldenummer: 99102198.1
(22) Anmeldetag: 04.02.1999
(51) Int. Cl.: C07D 313/04, C07D 315/00

(54) **Verfahren zur Herstellung von Lactonen**

(30) Priorität: 03.03.1998 DE 19808846
(71) Anmelder: HÜLS AKTIENGESELLSCHAFT, 45764 Marl (DE)
(72) Erfinder: Feld, Marcel, Dr., 51147 Köln (DE); Köhler, Günther, Dr., 45770 Marl (DE); Korell, Michael, Dr., 44801 Bochum (DE)
(74) Vertreter: Olbricht, Gerhard, Dr.

(57) **Zusammenfassung**

Die Erfindung betrifft ein Verfahren zur Herstellung von Lactonen der allgemeinen Formel I 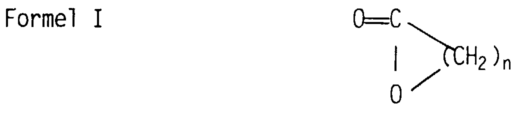 in der n die Bedeutung einer ganzen Zahl >2 hat, bei dem man omega-Hydroxycarbonsäuren oder deren Ester in monomerer, oligomerer oder polymerer Form der allgemeinen Formel II 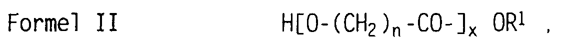 in der n die angegebenen Bedeutung hat, R¹ Wasserstoff oder einen Alkylrest, vorzugsweise mit 1 bis 4 Kohlenstoffatomen, bedeutet und x für eine ganze Zahl steht; bei 0.1 bis 1 mbar und 150 bis 300°C in ein einen gelösten Katalysator enthaltendes, unter den Reaktionsbedingungen inertes, hinreichend hochsiedendes Reaktionsmedium einträgt und das dampfförmig abdestillierende Lacton I durch Kondensation gewinnt.

## Beschreibung

Die Erfindung betrifft ein neues Verfahren zur Herstellung vorzugsweise 12- bis 20-gliedriger Lactone der allgemeinen Formel I 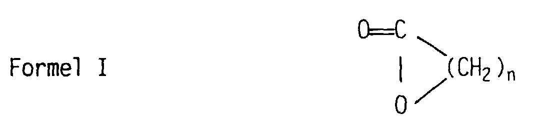 in der n die Bedeutung einer ganzen Zahl >2 hat, ausgehend von omega-Hydroxycarbonsäuren oder deren Estern in monomerer, oligomerer oder polymerer Form der Formel II 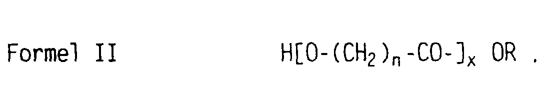 in der n die angegebenen Bedeutung hat, R Wasserstoff oder einen Alkylrest bedeutet und x für eine ganze Zahl steht.

Lactone sind als intramolekulare Ester von Hydroxycarbonsäuren potentiell bifunktionelle Moleküle und daher wertvolle Zwischenprodukte für eine Reihe von Synthesen. Makrocyclische Lactone mit 12 bis bis 20 Ringgliedern und insbesondere das Cyclopentadecanolid haben in der Parfümindustrie als Duftbestandteil oder Fixateur in Riechstoffmischungen eine herausragende Bedeutung.

Die Herstellung von Lactonen aus omega-Hydroxycarbonsäuren oder deren Estern durch intramolekulare, cyclisierende Veresterung oder Umesterung wird dadurch erschwert, daß sich unter den Verfahrensbedingungen auch lineare oligomere und polymere Ester bilden können. Dies vermindert nicht nur die Ausbeute an Zielprodukt, sondern wirft bei der Übertragung des Verfahrens in den technischen Maßstab, insbesondere bei einer batchweisen oder kontinuierlichen Reaktionsführung im Rührkessel, beträchtliche Probleme auf, Durch die gebildeten höhermolekularen Produkte sinkt nämlich die Wärmeleitfähigkeit des Reaktionsaemisches, während gleichzeitig die Viskosität ansteigt. Dadurch wird das Abdestillieren der monomeren Zielprodukte erschwert, was wiederum die Bildung höhermolekularer Produkte begünstigt. Deren Anteil steigt also an, wodurch in zunehmendem Maße Sumpfprodukt entsteht, das entsorgt werden muß. Wird die Reaktion nicht rechtzeitig abgebrochen, ist sogar ein Erstarren des gesamten Kesselinhalts möglich.

Die genannten Probleme werden durch das erfindungsgemäße Verfahren auf einfache, vorteilhafte und technisch leicht realisierbare Weise gelöst. Dabei wird nicht nur die Bildung höhermolekularer linearer Ester weitgehend unterdrückt. Das neue Verfahren läßt vielmehr sogar deren Verwendung als Ausgangsstoffe zu. indem sie durch Depolymerisation ebenfalls Lactone ergeben.

Gegenstand der Erfindung ist ein Verfahren zur Herstellung von Lactonen der allgemeinen Formel I 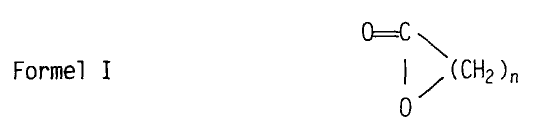
in der n die Bedeutung einer ganzen Zahl >2, vorteilhaft >5 und insbesondere >10 hat,
bei dem man omega-Hydroxycarbonsäuren oder deren Ester in monomerer. oligomerer oder polymerer Form der allgemeinen Formel II 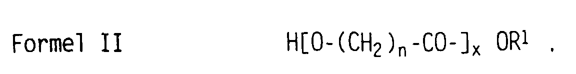
in der n die angegebenen Bedeutung hat, R¹ Wasserstoff oder einen Alkylrest, vorzugsweise mit 1 bis 4 Kohlenstoffatomen, bedeutet und x für eine ganze Zahl steht:
bei 0.1 mbar bis 1 bar und 150 bis 300°C, vorzugsweise bei 200 bis 280°C, in ein einen gelösten Katalysator enthaltendes, unter den Reaktionsbedingungen inertes, hochsiedendes Reaktionsmedium einträgt und das dampfförmig abdestillierende Lacton I durch Kondensation gewinnt.

Das erfindungsgemäße Verfahren eignet sich besonders zur Herstellung von makrocyclischen Lactonen mit 12 bis 20 Ringgliedern. In diesen Fällen steht also n für eine ganze Zahl von 10 bis 18.

Bezogen auf das Edukt II ist das erfindungsgemäße Verfahren kontinuierlich. Das Edukt kann gleichmäßig oder portionsweise und über Zeiträume von mehreren Tagen oder von Wochen zugeführt werden, ohne daß sich im Reaktionsmedium zu große Mengen an hochmolekularen Polymerprodukten ansammeln.

Die Edukte II sind offenkettige Moleküle mit Hydroxyl- und Carboxyl- oder Carbonesterfunktion, aus dem die cyclischen Lactone I sowie Wasser und/oder Alkohol ROH entstehen. Im einfachsten Fall ist das Edukt II eine omega-Hydroxycarbonsäure. Andere gut geeignete Edukte II lassen sich beispielsweise durch katalytische Oligomerisierung und gegebenenfalls Veresterung der omega-Hydroxycarbonsäuren oder durch katalytische Depolymerisation von hochpolymeren Poly-omega hydroxycarbonsäuren oder -estern (x z.B. ≥ ca. 100) in Gegenwart von Wasser und/oder Alkohol herstellen. Als Katalysatoren eignen sich die später beschriebenen Katalysatoren für das erfindungsgemäße Verfahren. In beiden Fällen bestimmt das molare Verhältnis von Wasser und/oder Alkohol zu omega-Hydroxycarbonsäure bzw. omega-Hydroxycarbonsäure-Baustein den Oligomerisierungs- bzw. Polymerisationsgrad x des Edukts II. Dabei handelt es sich um einen Mittelwert, da auf die beschriebene Weise Gemische von Molekülen mit unterschiedlichen Werten von x entstehen. In bevorzugten Edukten II bedeutet x eine Zahl ganze Zahl von bis zu etwa 50. Geeignete monomere Edukte II sind z.B. 4-Hydroxybuttersäure, 6-Hydroxycapronsäure, 8-Hydroxyoctansäure, 10-Hydroxydecansäure, 12-Hydroxydodecansäure, 14-Hydroxytetradecansäure, 15-Hydroxypentadecansäure und 18-Hydroxyoctadecansäure sowie deren Methyl- oder Ethylester und Oligomere oder Polymere dieser omega-Hydroxycarbonsäuren oder deren Ester mit Alkanolen mit 1 bis 6 Kohlenstoffatomen.

Als Katalysatoren können die üblichen sauren oder basischen Veresterungskatalysatoren verwendet werden. Bevorzugt werden Katalysatoren vom Typ einer Lewis-Säure, beispielsweise die in der gleichzeitig anhängigen deutschen Patentanmeldung P 198 08 845.0 beschriebenen Eisen(III)-Komplexe oder Salze des Eisens, Magnesiums, Mangans, Cadmiums, Cobalts, Zinns, Zinks, Bleis, Aluminiums oder Titans. Die Menge des Katalysators kann in weiten Grenzen variiert werden. Bei einer kontinuierlichen Ausführung des erfindungsgemäßen Verfahrens mit hinreichend langer Reaktionsperiode ergeben selbst hohe Konzentrationen, beispielsweise von 10 Gew. -%, bezogen auf das Reaktionsmedium, nur geringe Einsatzmengen an Katalysator, bezogen auf umgesetztes Edukt II. Wenn man, wie zuvor beschrieben, die Edukte II durch Oligomerisierung und gegebenenfalls Veresterung von omega-Hydroxycarbonsäuren oder durch Depolymerisation von Polyhydroxycarbonsäure oder -ester herstellt, enthalten die Reaktionsgemische bereits Katalysator, der für die Cyclisierung zum Lacton I geeignet ist.

Das Reaktionsmedium ist unter den Reaktionsbedingungen inert, enthält also keine Gruppen oder Atome, die mit dem Edukt II oder dem Zielprodukt I reagieren, und ist auch thermisch stabil, Sein Siedepunkt unter dem Reaktionsdruck liegt höher als der des Edukts II und des Zielprodukts I. Als geeignete Reaktionsmedien haben sich vor allem Polyalkylenglykoldiether erwiesen, vorzugsweise Polyalkylenglykoldiether mit einer Molmasse von ca. 1.000 und einem Endgruppenverschluß durch Alkylgruppen.

Bei einer möglichen Ausführungsform des erfindungsgemäßen Verfahrens wird das Edukt II gleichzeitig mit oder in Form einer Lösung in einem vergleichsweise leicht verdampfenden Lösungsmittel in das Reaktionsmedium eingetragen, das den Katalysator enthält, Überraschenderweise können dabei auch niedermolekulare Alkohole, z.B. Glykole, Alkanole oder Alkyoxyalkanole mit bis zu 6 Kohlenstoffatomen, mitverwendet werden, obwohl diese mit den Lactonen I unter dem Einfluß der eingesetzten Katalysatoren zu offenkettigen, im Falle von Glykolen auch oligomeren oder polymeren Estern zu reagieren vermögen. Geeignete niedermolekulare Alkohole sind z.B. Methanol, Ethanol, Propanol, Butanol, Methylglykol, Ethylglykol, Ethylenglykol und 1,2-Propylenglykol, Das vergleichsweise leicht verdampfende Lösungsmittel wird gegebenenfalls in einer Menge angewandt, die ausreicht, um das Edukt II zu lösen. Die Lactone I und Wasser oder die Alkoholkomponente des Edukts II sowie gegebenenfalls der niedermolekulare Alkohol destillieren ab. Der niedermolekulare Alkohol kann gegebenfalls durch Destillation von den Lactonen I abgetrennt werden.

In einer bevorzugten Form wird das erfindungsgemäße Verfahren so durchgeführt, daß das den Katalysator enthaltende Reaktionsmedium im Kreis über einen großoberflächigen Verdampfer geführt wird, von dessen Oberfläche das Lacton I sowie gegebenenfalls Wasser und/oder Alkohol abdestillieren. Das Edukt II und gegebenenfalls ein niedermolekularer Alkohol oder ein anderes leicht verdampfendes Lösungsmittel können dem Reaktionsmedium jeweils vor dem Eintritt in den Verdampfer zugesetzt werden. Optimale Mengenverhältnisse von Edukt II zum Reaktionsmedium, die einen nahezu vollständigen Umsatz des Edukts zum Lacton I ermöglichen, können bei konstanter Zufuhr von Edukt II durch Veränderung der Umlaufgeschwindigkeit des Reaktionsmediums oder bei konstanter Umlaufgeschwindigkeit des Reaktionsmediums durch veränderte Edukt-Zuführung eingestellt werden. Natürlich kann man auch durch Veränderung beider genannten Parameter regeln. Bevorzugt werden die Bedingungen so eingestellt, daß 1 kg Edukt II mit 0,1 bis 20, vorzugsweise mit 1 bis 15 und insbesondere mit 2 bis 10 kg Reaktionsmedium verdünnt werden, Ein größerer Verdünnungsgrad, also eine mehr als 20-fache Gewichtsmenge an Reaktionsmedium, bringt keinen meßbaren ökonomischen Vorteil, ist allerdings für den Reaktionsverlauf auch nicht nachteilig.

Als Verdampfer und zugleich Reaktoren eignen sich alle üblichen Verdampfer mit großer Oberfläche, wie Dünnschicht-, Fallfilm-, Rieselfilm- und Kurzwegverdampfer. Die erforderliche Wärmemenge kann in diesen Fällen vorteilhaft direkt über den Verdampfer eingebracht verden. Wird ein Kreislaufstrom des inerten hochsiedenden Mediums über einen Wärmetauscher geführt, so kann die Reaktion auch in einem Rieselbettreaktor stattfinden. Eine andere geeignete Variante ist ein Rührreaktor mit einem Festbettkatalysator, der mit einem üblichen Verdampfer mit großer Oberfläche über eine Kreislaufführung verburden ist. Schließlich kann man auch das vorerhitzte, Edukt II sowie Katalysator enthaltende hochsiedende Reaktionsmedium in einen Verdampfer einsprühen, in dem ein erhitztes inertes Trägergas strömt und das Lacton I austrägt, während das hochsiedende Reaktionsmedium mit dem Katalysator aus dem Verdampfer flüssig abläuft. In geeigneten Verdampfern liegt das hochsiedende Reaktionsmedium, welches Edukt II sowie Katalysator und nach Fortschreiten der Reaktion auch Lacton I enthält, in dünner Schicht von weniger als 2 cm Dicke, vorteilhaft von weniger als 0,5 cm Dicke oder in Tropfenform vor, bietet also eine große, verdampfungsfördernde spezifische Oberfläche. Dementsprechend kurz sind die Verweilzeiten des hochsiedenden Reaktionsmediums, in dem sich die Umwandlung vom Edukt zum Produkt vollzieht. Großoberflächige Verdampfer gestatten daher bei für das jeweilige Lacton I geeigneten Reaktionstemperaturen einen mindestens 80-prozentigen, vorteilhaft mindestens 90-prozentigen Umsatz des Edukts innerhalb einer Verweilzeit von weniger als 5 Minuten, vorteilhaft von weniger als 2 Minuten. Meistens liegen die Verweilzeiten sogar im Sekundenbereich.

Die aus dem Verdampfer austretenden Dämpfe werden in üblicher Weise kondensiert,und aus dem Destillat können die Lactone I durch Destillation, gegebenenfalls über eine Kolonne, gewonnen werden.

Wie zuvor erwähnt, lassen sich hochpolymere omega-Hydroxycarbonsäuren oder -ester (x ≥ ca. 100) in Gegenwart von Wasser und/oder Alkohol zu Edukten II mit durchschnittlichem Polymerisationsgraden x bis zu etwa 50 depolymerisieren, die sich für das erfindungsgemäße Verfahren besonders gut eignen, Dazu können, wie erwähnt, die gleichen Katalysatoren eingesetzt werden, die sich auch für die erfindungsgemäße Herstellung von Lactonen eignen. In diese Depolymerisation kann man auch einen Teil des im Kreis geführten Reaktionsmediums einbringen. Auf diese Weise werden etwa angereicherte hochpolymere Edukte II ebenfalls in besser geeignete Edukte II mit niedrigeren Polymerisationsgraden umgewandelt.

Die folgenden Beispiele sollen die Erfindung weiter erläutern, nicht aber ihren Anwendungsbereich begrenzen.

### Beispiel 1

In einem Rührreaktor mit absteigendem Kühler und Destillationsvorlage legt man 300 g Polyethylenglykoldimethylether (mittleres Molekulargewicht ca. 2000) und 0,5 g Mononatriumsalz des Eisen(III)-Komplexes der Ethylendiamintetraessigsäure vor. Die Mischung wird bei einem Druck von 1,0 mbar auf 260°C erhitzt. Pro Stunde werden nun 35 g eines Poly-epsilon-Caprolactons (mittleres Molekulargewicht ca. 10000) in die erhitzte Mischung eingebracht. Pro Stunde werden 34 bis 35 g Destillat gewonnen, das nach GC-Analyse 96,4% monomeres und 2,4% dimeres epsilon-Caprolacton enthält.

### Beispiel 2

In einem Ruhrreaktor mit absteigendem Kühler und Destillationsvorlage legt man 300 g Polyethylenglykoldimethylether (mittleres Molekulargewicht ca. 2000) und 0,5 g Mononatriumsalz des Eisen-(III)-Komplexes der Ethylendiamintetraessigsäure vor. Die Mischung wird bei einem Druck von 1,0 mbar auf 260°C erhitzt. Pro Stunde werden nun 20 g 15-Hydroxypentadecansäure-n-butylester im Gemisch mit 80 g n-Butanol in die erhitzte Mischung eingebracht. Pro Stunde werden etwa 98 g Destillat gewonnen. Nach Abdestillieren des n-Butanols bleiben pro Stunde ca 17 g 15-Hydroxypentadecansäurelacton (Cyclopentadecanolid) mit einer durch GC-Analyse bestimmten Reinheit von 95%.

## Patentansprüche

1. Verfahren zur Herstellung von Lactonen der allgemeinen Formel I 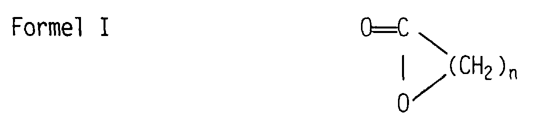
in der n die Bedeutung einer ganzen Zahl >2 hat,
bei dem man omega-Hydroxycarbonsäuren oder deren Ester in monomerer. oligomerer oder polymerer Form der allgemeinen Formel II 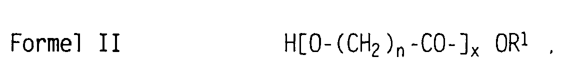
in der n die angegebenen Bedeutung hat. R¹ Wasserstoff oder einen Alkylrest, vorzugsweise mit 1 bis 4 Kohlenstoffatomen, bedeutet und x für eine ganze Zahl steht;
bei 0.1 bis 1 mbar und 150 bis 300°C in ein einen gelösten Katalysator enthaltendes, unter den Reaktionsbedingungen inertes, hinreichend hochsiedendes Reaktionsmedium einträgt und das dampfförmig abdestillierende Lacton I durch Kondensation gewinnt.

2. Verfahren nach Anspruch 1, dadurch gekennzeichnet, daß n in den Formeln I und II eine ganze Zahl >5 bedeutet.

3. Verfahren nach Anspruch 1, dadurch gekennzeichnet, daß n in den Formeln I und II eine ganze Zahl >10 bedeutet.

4. Verfahren nach Anspruch 1, dadurch gekennzeichnet, daß n in den Formeln I und II 10 bis 18 bedeutet, so daß die Lactone I 12 bis 20 Ringglieder haben.

5. Verfahren nach einem der Ansprüche 1 bis 4, dadurch gekennzeichnet, daß als inertes hinreichend hochsiedendes Reaktionsmedium Polyalkylenglykoldiether mit einer Molmasse von ca. 2.000 und einem Endgruppenverschluß durch Alkylgruppen verwendet wird.

6. Verfahren nach einem der Ansprüche 1 bis 5, dadurch gekennzeichnet, daß als Katalysator ein üblicher saurer oder basischer Veresterungskatalysator verwendet wird.

7. Verfahren nach der Anspruch 6, dadurch gekennzeichnet, daß als Katalysator vom Typ einer Lewis-Säure ein Eisen(III)-Komplex oder ein Salz des Eisens, Magnesiums, Mangans, Cadmiums, Cobalts, Zinns, Zinks, Bleis, Aluminiums oder Titans verwendet wird.

8. Verfahren nach einem der Ansprüche 1 bis 7, dadurch gekennzeichnet, daß man das erfindungsgemäße Verfahren durchführt, indem das den Katalysator enthaltende Reaktionsmedium im Kreis über einen großoberflächigen Verdampfer geführt wird, von dessen Oberfläche das Lacton I sowie Wasser und/oder Alkohol abdestillieren.

9. Verfahren nach der Anspruch 8, dadurch gekennzeichnet, daß als großoberflachiger Verdampfer ein Dünnschicht-, Fallfilm- oder Kurzwegverdampfer verwendet wird.

10. Verfahren nach Anspruch 8, dadurch gekennzeichnet, daß das Reaktionsmedium über einen Wärmetauscher und eine Rieselbettkolonne als großoberflächigen Verdampfer geführt wird.

11. Verfahren nach einem der Ansprüche 8 bis 10, dadurch gekennzeichnet, daß dem Verdampfer bis zu 50 kg Edukt II pro m² Verdampferoberfläche und Stunde zugeführt werden, die mit 0.1 bis 20 kg Reaktionsmedium pro kg Edukt II verdünnt werden.

12. Verfahren nach Anspruch 11, dadurch gekennzeichnet, daß die Verdünnung mit 1 bis 15 kg Reaktionsmedium pro kg Edukt II erfolgt.

13. Verfahren nach Anspruch 11, dadurch gekennzeichnet, daß die Verdünnung mit 2 bis 10 kg Reaktionsmedium pro kg Edukt II erfolgt.

14. Verfahren nach einem der Ansprüche 8 bis 13, dadurch gekennzeichnet, daß das Edukt II gleichzeitig mit oder in Form einer Lösung in einem vergleichsweise leicht verdampfenden Lösungsmittel in das Reaktionsmedium eingetragen wird.

15. Verfahren nach einem der Ansprüche 1 bis 14, dadurch gekennzeichnet, daß hochpolymere omega-Hydroxycarbonsäuren oder -ester zunächst in Gegenwart von Wasser und/oder Alkohol depolymerisiert und dann als Edukt II eingesetzt werden.
